# EUROPÄISCHE PATENTSCHRIFT

(11) **EP 1 610 125 B1**
(45) Veröffentlichungstag und Bekanntmachung des Hinweises auf die Patenterteilung: **12.12.2007**
(21) Anmeldenummer: 05104830.4
(22) Anmeldetag: 03.06.2005
(51) Int. Cl.: G01N 33/28, G01N 25/08, F02D 19/06

(54) **Verfahren sowie Einrichtung zur Bestimmung der Verdampfungseigenschaft von Kraftstoff-Flüssigkeiten**
Apparatus and method for determining the vaporization characteristic of fluif fuels
Dispositif et procédé pour déterminer la characteristic de vaporisation de carburants fluides

(30) Priorität: 25.06.2004 DE 102004030729
(43) Veröffentlichungstag der Anmeldung: 28.12.2005
(73) Patentinhaber: Hella KGaA Hueck & Co., 59552 Lippstadt (DE)
(72) Erfinder: Müller, Hagen, 33181, Bad Wünnenberg (DE); Kühnau, Uwe, 59227, Ahlen (DE)

(56) Entgegenhaltungen:
- US-A- 5 054 460
- US-A1- 2003 005 754
- US-B1- 6 499 476
- PATENT ABSTRACTS OF JAPAN Bd. 1999, Nr. 04, 30. April 1999 (1999-04-30) & JP 11 013568 A (TOYOTA MOTOR CORP), 19. Januar 1999 (1999-01-19)
- PATENT ABSTRACTS OF JAPAN Bd. 1995, Nr. 11, 26. Dezember 1995 (1995-12-26) & JP 07 198710 A (HONDA MOTOR CO LTD), 1. August 1995 (1995-08-01)

## Beschreibung

Die Erfmdung bezieht sich auf ein Verfahren zur Bestimmung der Verdampfungseigenschaft von Kraftstoff-Flüssigkeiten für Verbrennungsmotoren von Kraftfahrzeugen sowie auf eine Einrichtung zur Bestimmung der Verdampfungseigenschaft von Kraftstoff-Flüssigkeiten.

Für einen zuverlässigen Start eines Ottomotors ist eine ausreichende Menge an verdampftem Kraftstoff im Verbrennungsraum notwendig, um ein zündfähiges Kraftstoff/Luftgemisch zu erhalten. Nun sind jedoch die Verdampfungseigenschaften von Ottokraftstoffen, insbesondere die von Ottokraftstoffen mit Sommerqualität oder Winterqualität sehr unterschiedlich, wobei die Verdampfungseigenschaften von Kraftstoffen anhand des Dampfdruckes nach Reid spezifiziert werden. Zum Beispiel liegt der gemäß der Norm EN 228 zulässige Dampfdruck für einen Ottokraftstoff mit Sommerqualität zwischen 45 und 60 kPa (KiloPascal), während der gemäß der Norm EN 228 zulässige Dampfdruck für einen Ottokraftstoff mit Winterqualität zwischen 60 und 95 kPa liegt. Die Dampfdruckwerte von Ottokraftstoffen nach Reid, d.h. die Verdampfungseigenschaften, und damit auch die Zündfähigkeit des mit dem jeweiligen Kraftstoff erzielten Kraftstoff/Luftgemisches variieren über einen sehr großen Bereich von 45kPa bis 95kPa.

Aus Unkenntnis über die Verdampfungseigenschaften des jeweils verwendeten (getankten) Kraftstoffs wird die für einen zuverlässigen Start notwendige Kraftstoffmenge sehr stark überdosiert. Dies führt zu einem Mehrverbrauch an Kraftstoff und zu stark verschlechterten Abgaswerten in der Startphase.

Das mit der Unkenntnis der unterschiedlichen Kraftstoffeigenschaften verbundene Problem beim Startvorgang wird beispielsweise in der DE 199 55 796 A1 angesprochen. Dabei wird in der DE 199 55 796 A1 zur Lösung des Problems die Verwendung einer Kraftstoffbeurteilungsvorrichtung vorgeschlagen, um anhand der beurteilten Kraftstoffeigenschaften eine genau an die jeweiligen Kraftstoffeigenschaften angepasste Kraftstoffdosierung vornehmen zu können. Dabei wird in der DE 199 55 796 A1 unter anderem ein Kapazitätssensor zur Bestimmung der Dielektrizitätszahl des Kraftstoffs und ein optischer Sensor zur Bestimmung von optischen Kraftstoffeigenschaften als Kraftstoffbeurteilungsvorrichtung genannt. Eine unmittelbare Bestimmung der Verdampfungseigenschaften von Kraftstoffen ist damit jedoch nicht möglich. Aus der JP-07198710 ist ein weiteres Beispiel für die Bestimmung der Verdampfungseigenschaft von Kraftstoff-Flüssigkeiten bekannt.

Aufgabe der Erfmdung ist es, ein Verfahren und eine Einrichtung zu schaffen, mit dem unmittelbar eine Bestimmung der Verdampfungseigenschaften von Kraftstoffen ermöglicht wird.

Zur Lösung dieser Aufgabe wird erfindungsgemäß ein elektrisches Heizelement verwendet, das zumindest teilweise in der Kraftstoff-Flüssigkeit angeordnet ist, so daß eine Wärmeleitung zwischen dem Heizelement und der Kraftstoff-Flüssigkeit gewährleistet ist. Dabei wird das Heizelement zumindest bis zur Bildung von Kraftstoffdampfblasen an der Heizelement-Oberfläche aufgeheizt, wobei die Temperatur an oder zumindest in unmittelbarer Nähe des Heizelements beim Aufheizen gemessen wird. Zur Charakterisierung der Verdampfungseigenschaften des Kraftstoffs wird die Siedetemperatur oder, da es sich bei Kraftstoffen in der Regel um Flüssigkeitsgemische handelt, die Anfangssiedetemperatur des Siedebereichs oder gar die gesamte Siedelinie des Kraftstoffs ermittelt. Dies geschieht, indem die sich lokal am Heizelement bildenden Dampfblasen detektiert werden, wobei insbesondere der Einfluß des dampfblaseninduzierten konvektiven Wärmetransports zwischen Heizelement und Kraftstoff-Flüssigkeit auf den Temperaturverlauf ermittelt wird.

Durch das Aufheizen des Heizelements wird eine Temperaturerhöhung am Heizelement und ein Wärmetransport vom Heizelement in die Kraftstoff-Flüssigkeit bewirkt. Dadurch wird das Heizelement gekühlt und die das Heizelement umgebende Kraftstoff-Flüssigkeit lokal erwärmt, wobei der Wärmetransport vom Heizelement in die Kraftstoff-Flüssigkeit vor der Dampfblasenbildung von konduktiver Art ist, ähnlich wie der Wärmetransport innerhalb von Festkörpern. Bei weiterem Aufheizen wird am Heizelement und in der Kraftstoff-Flüssigkeit lokal, d.h. in unmittelbarer Nähe des Heizelements, die Siedetemperatur bzw. die Anfangssiedetemperatur des Kraftstoffs erreicht. Dies führt lokal am Heizelement zur Bildung von Dampfblasen (Blasensieden). Diese Dampfblasen steigen in der Kraftstoff-Flüssigkeit auf, wobei in der Wirbelschleppe der aufsteigenden Dampfblasen kühlere Kraftstoff-Flüssigkeit zum Heizelement strömt. Der dampfblaseninduzierte konvektive Wärmetransport führt zu einem wesentlich verbesserten Wärmeübergang vom Heizelement in die Kraftstoff-Flüssigkeit, d.h. zu einer effektiveren Kühlung des Heizelements durch die dampfblaseninduzierte Strömung. Bei Beginn der Dampfblasenbildung ändert sich der Temperaturverlauf am Heizelement signifikant, wobei die Temperatur am Heizelement nach dem Beginn der Dampfblasenbildung trotz weiterem Aufheizen zunächst annährend konstant bleibt oder zumindest wesentlich langsamer ansteigt als vor der Dampfblasenbildung. Das Verdampfungsverhalten des Kraftstoffs lässt sich somit über die Temperatur (Übergangstemperatur), bei welcher sich erste Dampfblasen bilden, charakterisieren. Der Einfluß der dampfblaseninduzierten Strömung auf den Temperaturverlauf am Heizelement ist besonders groß und kann daher zur Detektion von Dampfblasen in zuverlässiger Weise verwendet werden.

Die Einrichtung zur Bestimmung der Verdampfungseigenschaft von Kraftstoff-Flüssigkeiten für Verbrennungsmotoren von Kraftfahrzeugen ist gekennzeichnet durch einen mit Kraftstoff-Flüssigkeit gefüllten Meßraum, in dem das Heizelement und das Temperaturmeßmittel angeordnet sind, so daß diese vor störenden Strömungen, z.B. hervorgerufen durch die Förderung des Kraftstoffs durch die Kraftstoffpumpe, geschützt sind, so dass nur die Dampfblasenbildung eine Konvektion in einer ansonsten ruhenden Kraftstoff-Flüssigkeit hervorruft. Um die dampfblaseninduzierte Strömung zu optimieren, ist in dem Meßraum vorzugsweise eine Aufstromsäule und eine Abstromsäule realisiert, wobei zur Realisierung Aufstrom-/Abstromsäule ein Außenrohr und ein darin angeordnetes Innenrohr vorgesehen ist, in dem das Heizelement angeordnet ist.

Die Dampfblasendetektion kann erfindungsgemäß - alternativ zur Bestimmung des Einflusses der dampfblaseninduzierten Strömung auf den Temperaturverlauf - auch über ein kapazitives Sensorelement zur Messung der dielektrischen Eigenschaften des von dem Heizelement lokal aufgeheizten Kraftstoffs erfolgen, wobei sich der Umstand zunutze gemacht wird, dass sich die Dielektrizitätszahl in der Nähe des Heizelements bei Beginn der Dampfblasenbildung stark ändert.

Anhand der beigefügten Zeichnungen soll die Erfindung nachfolgend näher erläutert werden. Es zeigt:
- Figur 1: eine schematische Darstellung der Kraftstoffanlage eines Kraftfahrzeugs mit den potentiellen Messstellen, an denen das erfmdungsgemäße Verfahren durchgeführt werden kann,
- Figur 2: eine Schaltung mit einem temperaturabhängigen Widerstands-Heizelement, das gleichzeitig auch zur Temperaturmessung dient,
- Figur 3: ein Blockschaltbild zur Ansteuerung des Heizelements und zur Temperaturmessung,
- Figur 4: ein Blockschaltbild wie in Figur 3, jedoch unter Einbeziehung eines zusätzlichen kapazitiven Sensorelements,
- Figur 5: eine Übertemperaturschutzschaltung,
- Figur 6: eine Ansicht eines Messraums mit einer konzentrischen Anordnung von Außenrohr und Innenrohr zur Ausbildung einer Aufstromsäule und einer Abstromsäule,
- Figur 6A: eine Draufsicht auf eine konzentrische Anordnung von Außenrohr und Innenrohr zur Ausbildung von Aufstrom-/Abstromsäule,
- Figur 6B: eine Draufsicht auf eine nicht konzentrische Anordnung von Außenrohr und Innenrohr zur Ausbildung von Aufstrom-/Abstromsäule,
- Figur 6C: eine Ansicht einer nicht konzentrischen Anordnung von Außenrohr und Innenrohr zur Ausbildung von Aufstrom-/Abstromsäule,
- Figur 7: eine Ansicht eines Messraums mit einer konzentrischen Anordnung von Außenrohr und Innenrohr zur Ausbildung einer Aufstromsäule und einer Abstromsäule, wobei das Heizelement auf der Innenwandung des Innenrohres angeordnet ist,
- Figur 8: zeigt eine Anordnung bei der der Messraum in dem Abzweig eines T-förmigen Leitungsabschnitts der Kraftstoffleitung angeordnet ist, wobei der Abzweig des T-förmigen Leitungsabschnitts das Außenrohr der Abstromsäule bildet,

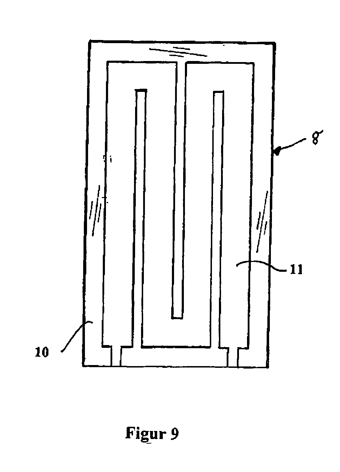

- Figur 9: eine stark vergrößerte Ansicht eines Heizelements, bei dem auf einem Keramiksubstrat eine Platin-Dünnschichtbahn als Widerstandsheizelement angeordnet ist,
- Figur 10: eine Anordnung bei der der Messraum in dem Abzweig eines T-förmigen Leitungsabschnitts der Kraftstoffleitung angeordnet ist, wobei die Anordnung aus Außenrohr und Innenrohr in den Abzweig des T-förmigen Leitungsabschnitts eingeschraubt wird,
- Figur 11: eine auf den Kopf gestellte Anordnung bestehend aus Außenrohr und Innenrohr, wie in Figur 10, bei der auf der Stirnseite des Außenrohrs ein Netz oder eine gelochte Platte angeordnet ist, um ein schnelles Herausfließen des Kraftstoffs aus dem das Heizelement aufweisenden Innenrohr zu verhindern,
- Figur 12: den Temperaturverlauf gegenüber der Zeit beim Aufheizen eines Ottkraftstoffs für drei jeweils verschiedene rampenförmig ansteigende Heizstrombelastungen des Heizelements,
- Figur 13: den Temperaturverlauf von Ottokraftstoff in Abhängigkeit von der dem Heizelement zugeführten Wärmemenge,
- Figur 14: den Temperaturverlauf von Ottokraftstoff mit Winterqualität in Abhängigkeit von der dem Heizelement zugeführten Wärmemenge,
- Figur 15: den Temperaturverlauf von Ottokraftstoff mit Sommerqualität in Abhängigkeit von der dem Heizelement zugeführten Wärmemenge,
- Figur 16: den Verlauf der Dielektrizitätszahl von Ottokraftstoff in Abhängigkeit der Temperatur.

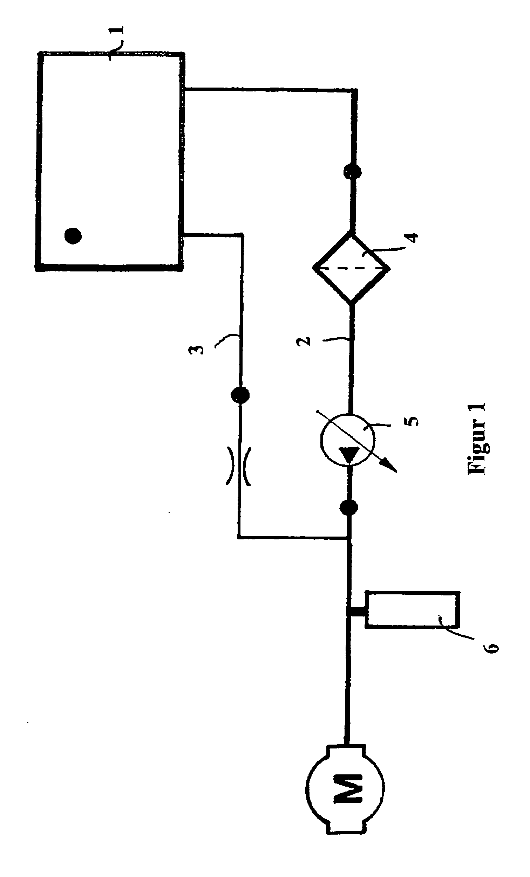

Figur 1 zeigt schematisch die Kraftstoffanlage eines Kraftfahrzeuges bestehend aus dem Kraftstofftank (1), der Kraftstoffzuführleitung (2) zum Verbrennungsmotor (M), dem Kraftstoff-Filter (4), der Kraftstoffpumpe (5) und einer Einspritzpumpe (6) sowie einer Kraftstoffrückleitung (3). Die möglichen Meßstellen zur Durchführung des Verfahrens sind durch Punkte markiert.

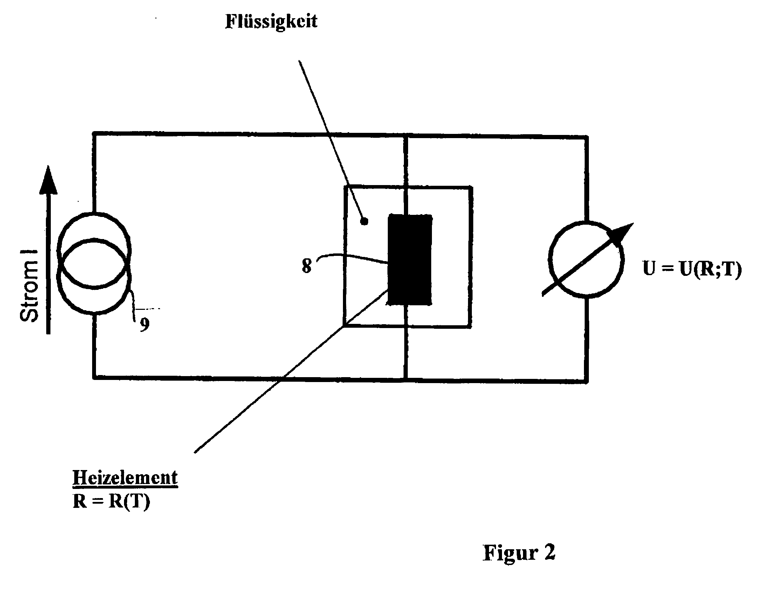

In Figur 2 ist eine Schaltung mit einem temperaturabhängigen Widerstand als Heizelement (8), das in der Kraftstoff-Flüssigkeit angeordnet ist, dargestellt, wobei das Heizelement (8) über eine steuerbare Stromquelle (9) mit Heizstrom beaufschlagt wird. Dabei wird als Widerstands-Heizelement (8) vorzugsweise eine auf einem Keramiksubstrat (10) angeordnete Platin-Dünnschichtbahn (11) verwendet. Die Temperaturmessung erfolgt dann über eine Bestimmung des temperaturabhängigen Widerstandes, so daß das Heizelement (8) selbst als Temperatursensor verwendet wird.

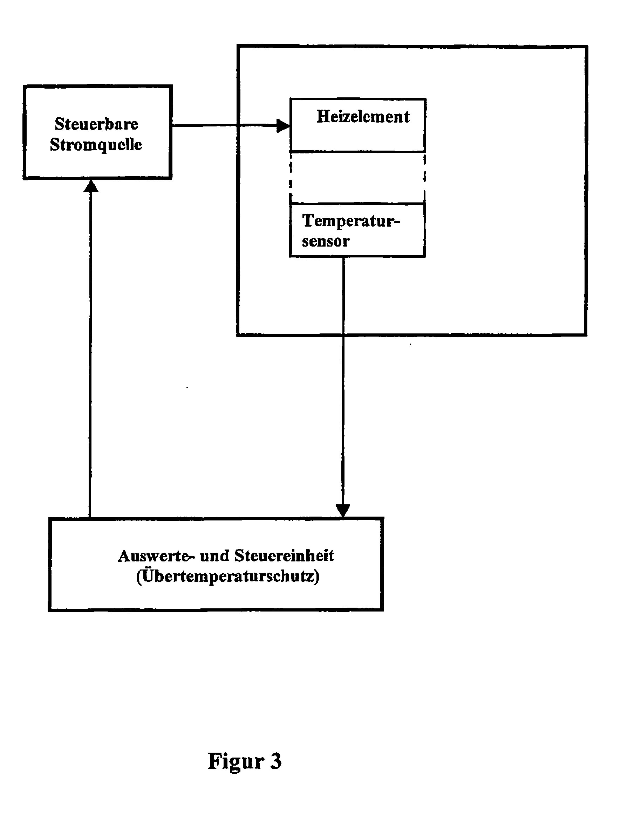

In Figur 3 ist eine Auswerte- und Steuereinheit dargestellt, welche einerseits die steuerbare Stromquelle für das Heizelement ansteuert und andererseits das Temperatursignal der Temperatur an oder zumindest in unmittelbarer Nähe des Heizelements empfängt und so den Temperaturverlauf ermittelt. Dabei ist es auch vorgesehen, einen vom Heizelement unabhängigen Temperatursensor zu verwenden. Das Aufheizen des Heizelements erfolgt in einer Ausführungsform, dadurch, dass das Heizelement mit einem zeitlich ansteigenden Strom, insbesondere einem linear ansteigenden Strom (Stromrampe), beaufschlagt wird. In einer weiteren Ausführungsform wird das Heizelement mit einem dreieckförmigen Stromsignal, d.h. einem linear ansteigenden und einem linear fallenden Stromsignal, beaufschlagt. In einer weiteren Ausführungsform erfolgt die Strombeaufschlagung des Heizelements, dadurch, dass dasselbe im Pulsbetrieb mit Strom beaufschlagt wird, wobei die Pulsweite der einzelnen Pulse und/oder die Stromhöhe der einzelnen Pulse variiert wird.

In vorteilhafter Weise erfolgt vor dem Aufheizen des Heizelements eine Temperaturmessung zur Bestimmung der Kraftstoff-Temperatur. Zu diesem Zweck kann das Heizelement selbst als Temperatursensor oder ein separater Temperatursensor verwendet werden.

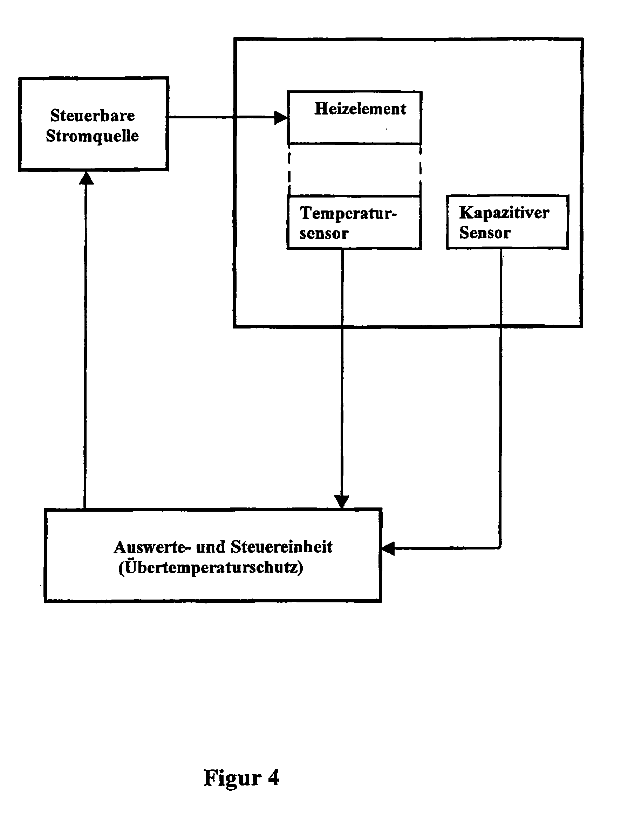

In Figur 4 ist eine Auswerte- und Steuereinheit gezeigt, welche zusätzlich das Signal eines kapazitiven Sensorelements zur Dampfblasendetektion empfängt. Das kapazitive Sensorelement ist am oder in der Nähe des Heizelements angeordnet, vorzugsweise ist das kapazitive Sensorelement auf demselben Substrat/Träger angeordnet wie das Heizelement. In einer Ausführungsform (nicht dargestellt) ist es vorgesehen, dass zumindest eine der Elektroden des kapazitiven Sensorelements gleichzeitig auch als Widerstandsheizelement verwendet wird.

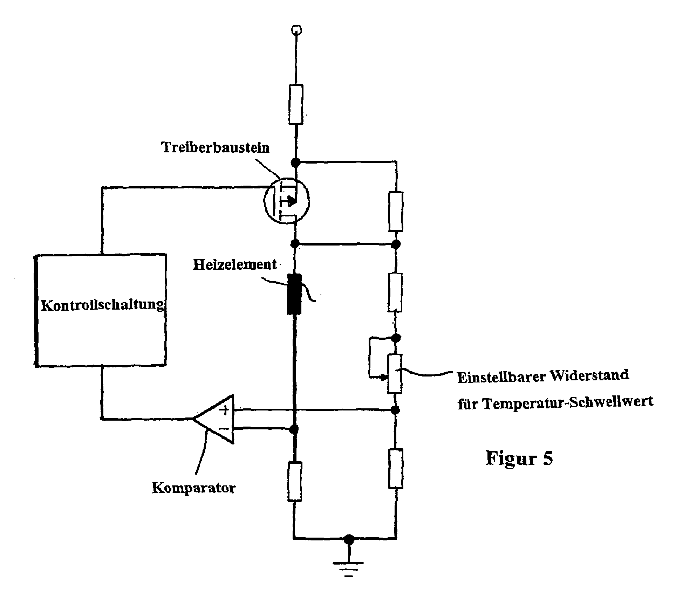

Falls das Heizelement (8) - aus welchem Grund auch immer - nicht oder nicht mehr von der das Heizelement (8) kühlenden Kraftstoff-Flüssigkeit umgeben sein sollte, kann es bei einem weiteren Aufheizen zu einer starken Überhitzung des Heizelements (8) kommen, wobei schnell Temperaturen am Heizelement (8) erreicht werden, die weit oberhalb des Siedebereichs des Kraftstoffs liegen. Bei Verwendung eines sogenannten Platin-Mikroheizers als Heizelement (8) können dabei leicht Temperaturen von bis zu 500° C erreicht werden. Da das Heizelement (8) innerhalb der Kraftstoffanlage angeordnet ist, besteht somit bei Anwesenheit von Luftsauerstoff die Gefahr der Entzündung des Kraftstoffs. Darüber hinaus kommt es bei derart hohen Temperaturen in unerwünschter Weise zu einem Cracken bzw. einer Verkokung des Kraftstoffs. Aus diesem Grunde ist zum Schutz vor einer Überhitzung in der Auswerte- und Steuereinheit eine Übertemperaturschutzschaltung vorgesehen, die die Stromzufuhr zum Heizelement (8) bei Erreichen einer vorbestimmten Temperatur (Temperaturschwelle) am Heizelement (8) verringert oder unterbindet. In Figur 5 ist eine Übertemperaturschutzschaltung gezeigt. Dabei wird der temperaturabhängige Spannungsabfall über dem Heizelement (8) mit dem Spannungsabfall in einem Referenzzweig über einen Komparator verglichen, wobei über einen verstellbaren Widerstand ein Temperaturschwellwert eingestellt werden kann. Wenn die Temperatur am Heizelement (8) diese Temperaturschwelle von z.B. 200°C, überschritten hat, ändert sich der Ausgang des Komparators, was wiederum von einer Kontrollschaltung erkannt wird, welche dann den Treiberbaustein für die Heizstromzufuhr schlagartig ausschaltet.

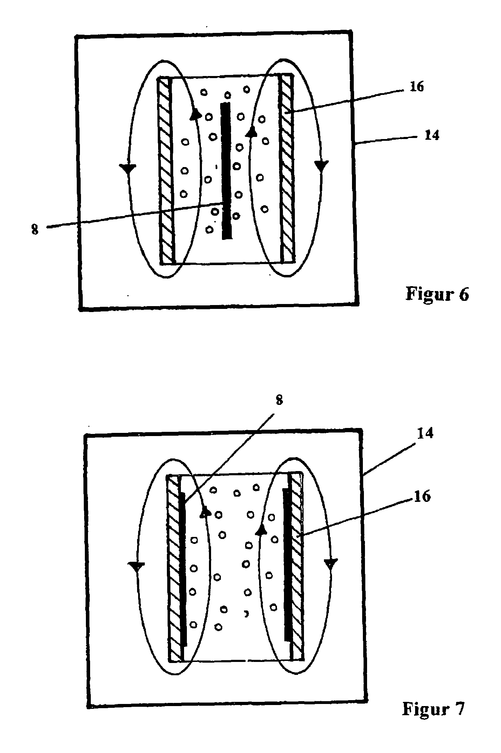

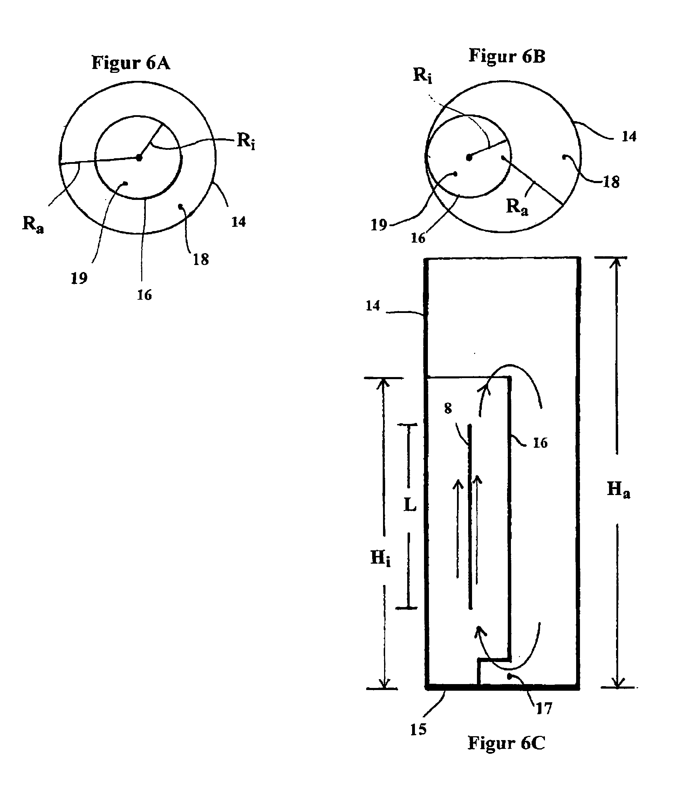

Figur 6 zeigt die Ansicht eines Messraums mit einer konzentrischen Anordnung von Außenrohr (14) und Innenrohr (16) zur Ausbildung einer Aufstromsäule und einer Abstromsäule, wobei das Heizelement (8) ebenfalls konzentrisch im Innenrohr angeordnet ist. Um ein Aufsteigen der sich bildenden Dampfblasen zu ermöglichen, sind die Längsachsen von Außenrohr (14) und Innenrohr (16) im wesentlichen lotrecht im Kraftfahrzeug angeordnet, wobei auch die Heizfläche des Heizelements (8) im wesentlichen parallel zum Lot angeordnet ist. Abweichungen von der lotrechten Lage von bis zu 30° sind dabei noch tolerierbar.

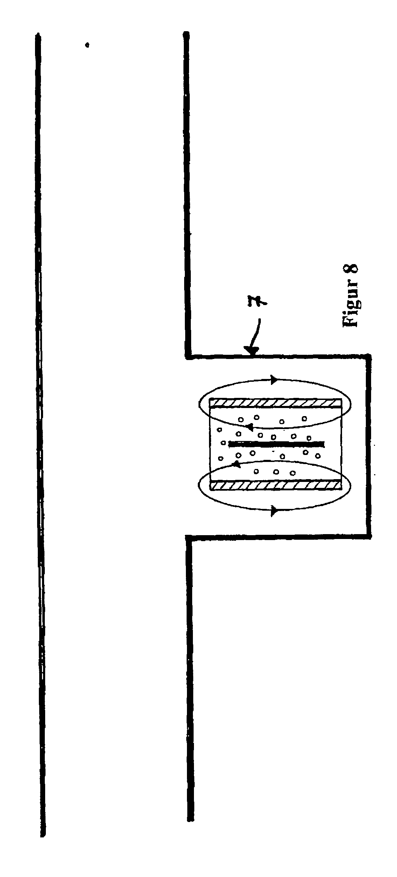

Figur 7 zeigt ebenfalls eine konzentrische Anordnung von Außenrohr (14) und Innenrohr (16), wobei in dieser Ausführungsform allerdings die Widerstandsbahnen des Heizelement (8) auf der Innenwandung des Innenrohrs (16) angeordnet sind. Diese Ausführungsform hat den Vorteil, dass eine Halterung zur Fixierung des Heizelements (8) im Innenrohr (16) entfällt.

Figur 6A zeigt eine Draufsicht auf die konzentrische Anordnung von Außenrohr (14) und Innenrohr (16), wobei Rₐ den Radius des Außenrohrs und Rᵢ den Radius des Innenrohrs bezeichnet. Um eine optimale dampfblaseninduzierte Strömung zu erreichen, sind vorzugsweise die folgenden geometrischen Verhältnisse realisiert:
- freie Querschnittsfläche (18) des Außenrohrs mindestens dreimal so groß ist wie die freie Querschnittsfläche (19) des Innenrohrs,
- Höhe (Hᵢ) des Innenrohrs mindestens eineinhalbmal so groß ist wie die Erstreckung (L) der Heizfläche des Heizelements (8) in Längsrichtung des Innenrohrs,
- Höhe (Hₐ) des Außenrohrs mindestens so groß ist wie die Höhe (Hᵢ) des Innenrohrs plus dem freien Durchmesser des Innenrohrs.

Figur 6B zeigt eine Draufsicht auf eine nicht konzentrische Anordnung von Außenrohr (14) und Innenrohr (16), bei der das Innenrohr am Außenrohr anliegt. Figur 6C zeigt eine entsprechende Ansicht dieser Anordnung in Längserstreckung des Außen- und Innenrohres. Im unteren Bereich des Innenrohres (16) ist eine Einströmöffnung (17) für in das Innenrohr nachströmende Kraftstoff-Flüssigkeit vorgesehen. Diese nicht konzentrische Anordnung hat den Vorteil, dass Haltestege zur Fixierung des Innenrohrs im Außenrohr entfallen.

Figur 8 zeigt eine Ausführungsform, bei der der Meßraum zum Schutz vor nicht dampfblaseninduzierten Strömungen in dem Abzweig (7) eines T-förmigen Leitungsabschnitts der Kraftstoffleitung angeordnet ist, wobei der Abzweig (7) des T-förmigen Leitungsabschnitts das Außenrohr der Aufström-/Abströmsäule bildet.

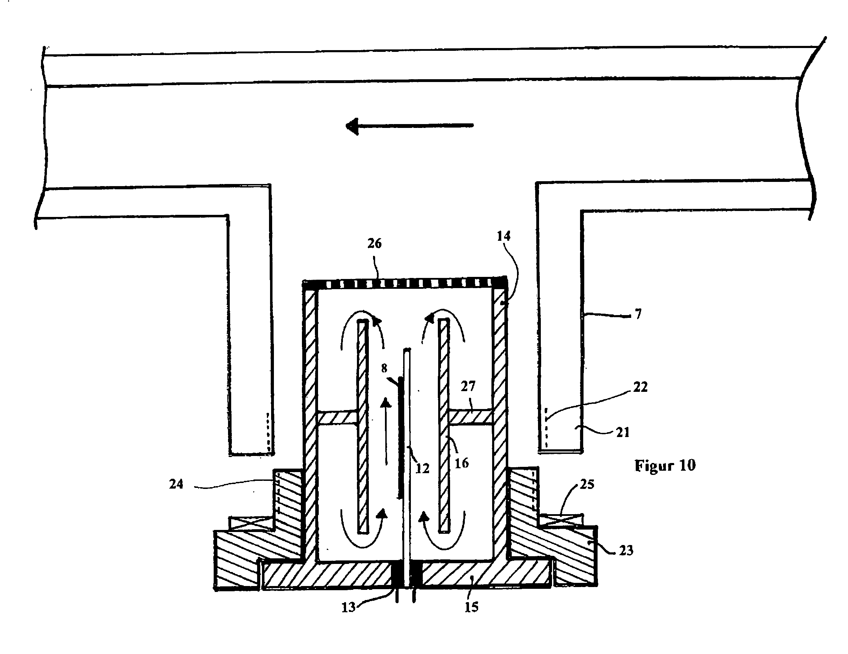

Figur 10 zeigt eine Ausführungsform, bei der der Meßraum ebenfalls in dem Abzweig (7) eines T-förmigen Leitungsabschnitts angeordnet ist. Allerdings wird hier die Anordnung aus Außenrohr (14) und Innenrohr (16) in die Rohrwandung (21) des Abzweigs (7) eingeschraubt. Zu diesem Zweck weist die erfindungsgemäße Einrichtung eine Grundplatte (23) mit einem Außengewinde (24) auf, das in das Innengewinde (22) der Rohrwandung (21) des T-förmigen Abzweigs (7) eingeschraubt wird. Von dieser Grundplatte (23) wird der Boden (15) des Außenrohrs (14) aufgenommen, wobei sich die Wandung des Außenrohrs (14) von diesem Boden (15) aus erstreckt. Das Innenrohr (16) wird über Haltestege (27) in dem Außenrohr (16) gehalten. Der Träger (12) für das Heizelement (8) wird über einen Stopfen (13), der abgedichtet im Boden des Außenrohrs angeordnet ist, gehalten. Der Boden (15) selbst liegt auf einer Stufe der Grundplatte (23) auf und ist dort z.B. mit dieser verklebt. Damit die Grundplatte (23) den T-förmigen Abzweig (7) druckdicht verschließt, weist die Grundplatte (23) auf einer weiteren Stufe eine umlaufende Dichtung (25) auf, die beim Einschrauben gegen die Stirnfläche der Rohrwandung (21) des T-förmigen Abzweigs (7) gepresst wird. Das Außenrohr (14), der Boden (15), das Innenrohr (16) sowie die Haltestege (27) für das Innenrohr (16) sind vorzugsweise einstückig aus Kunststoff hergestellt. Die Grundplatte (23) besteht aus Metall, vorzugsweise Aluminium.

Für den Fall, dass es trotz der Übertemperaturschutzschaltung dennoch zu einem Entflammen von Kraftstoff oder Kraftstoffdampf innerhalb des Innenrohres (16) kommt, weist zumindest die offene Stirnseite des Innenrohes (16) eine gelochte Metall-Platte/Metalldrahtnetz (26) auf. Dabei kühlt die Flamme sich am Metalldrahtnetz (26) so weit ab, dass sie nicht mehr heiß genug ist, um leicht entzündliche Kraftstoffdämpfe außerhalb des Metalldrahtnetzes (26) zu entzünden. Ein Effekt der bereits bei den frühen Sicherheits-Grubenlampen ausgenutzt wurde. In der Ausführungsform gemäß Figur 10 bedeckt das Metalldrahtnetz (26) das Außenrohr (14) und das Innenrohr (16). Der Durchmesser der Öffnungen im Metalldrahtnetz (26) beträgt ca. 0,5mm. Das Metalldrahtnetz ermöglicht somit einerseits aufgrund seiner Durchlässigkeit die strömungs- und turbulenzgedämpfte Zufuhr und den Austausch von Kraftstoff-Flüssigkeit und bewirkt andererseits einen effektiven Flammschutz.

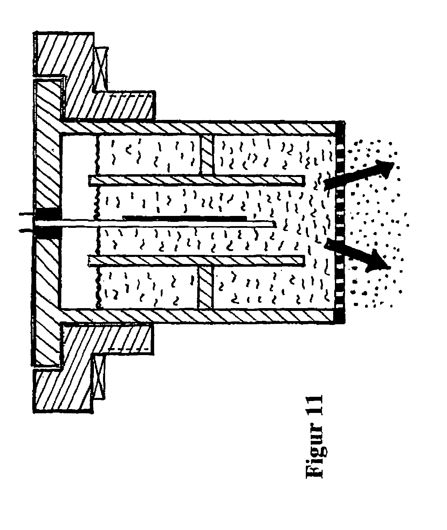

Infolge eines Unfalls kann es zu einem Fahrzeugüberschlag kommen, wobei dann auch die Anordnung bestehend aus Außenrohr (14) und Innenrohr (16) auf den Kopf gestellt wird - siehe Figur 11). Dies führt nun wiederum dazu, dass die Kraftstoff-Flüssigkeit aus dem Innenrohr herausfließt und das heiße Heizelement nicht mehr von der kühlenden Kraftstoff-Flüssigkeit umgeben ist. Stattdessen gelangt Luftsauerstoff an das heiße Heizelement und kann dort zu einer Entzündung des Kraftstoff führen. In dieser Situation verhindert das engmaschige Metalldrahtnetz (26) ein schnelles Herausfließen des Kraftstoffs aus dem das Heizelement enthaltenen Innenrohr. Auf diese Weise kann sichergestellt werden, dass das Heizelement nach einer Notabschaltung infolge eines Unfalls noch solange von der kühlenden und vor Sauerstoff schützenden Kraftstoff-Flüssigkeit umgeben ist bis die Temperatur des Heizelements bis auf einen unkritischen Wert gesunken ist. Als Mittel zum Drosseln des Querschnitts, um ein schnelles Herausfließen von Kraftstoff zu verhindern, muß das Drahtnetz selbstverständlich nicht aus Metall sein. Jedoch ist es vorteilhaft ein Metalldrahtnetz zu verwenden, da somit gleichzeitig auch der "Grubenlampen-Effekt " bewirkt wird.

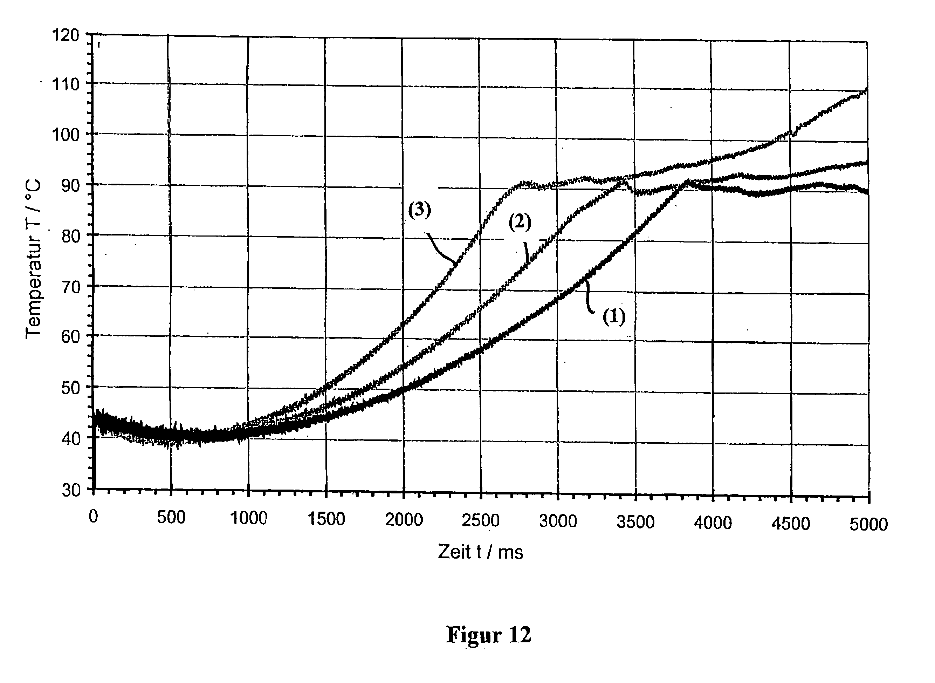

Figur 12 zeigt den zeitlichen Temperaturverlauf an einem in Ottkraftstoff eingetauchten Heizelement in einer erfmdungsgemäßen Aufstrom-/Abstromanordnung für drei verschiedene Heizkurven, wobei jeweils innerhalb einer Zeit von 0 bis 5 Sekunden ein rampenförmiger Anstieg des Heizstroms erfolgte:
Kurve (1): 100 - 550 mA
Kurve (2): 100 - 650 mA
Kurve (3): 100 - 800 mA.
Als Heizelement wurde dabei ein sogenannter Platin-Mikroheizer der Fa. Heraeus Sensor Technology verwendet.

Wie zu erkennen ist, nimmt die Temperatur am Heizelement zunächst mit steigendem Heizstrom zu, wobei der Anstieg natürlich von der Steigung der Heizstromrampe abhängt. Bei weiterem Aufheizen wird am Heizelement und in der Kraftstoff-Flüssigkeit lokal, d.h. in unmittelbarer Nähe des Heizelements, die Siedetemperatur bzw. die Anfangssiedetemperatur des Kraftstoffs erreicht. Dies führt lokal am Heizelement zur Bildung von Dampfblasen (Blasensieden). Der dampfblaseninduzierte konvektive Wärmetransport führt zu einem wesentlich verbesserten Wärmeübergang vom Heizelement in die Kraftstoff-Flüssigkeit, d.h. zu einer effektiveren Kühlung des Heizelements durch die dampfblaseninduzierte Strömung. Bei Beginn der Dampfblasenbildung ändert sich der Temperaturverlauf am Heizelement signifikant, wobei die Temperatur am Heizelement nach dem Beginn der Dampfblasenbildung trotz weiterem Aufheizen zunächst annährend konstant bleibt oder zumindest wesentlich langsamer ansteigt als vor der Dampfblasenbildung. Das Verdampfungsverhalten des Kraftstoffs lässt sich somit über die Temperatur (Übergangstemperatur), bei welcher sich erste Dampfblasen bilden, charakterisieren. Die Übergangstemperatur liegt bei dem in Figur 12 verwendeten Ottokraftstoff bei ca. 92°C.

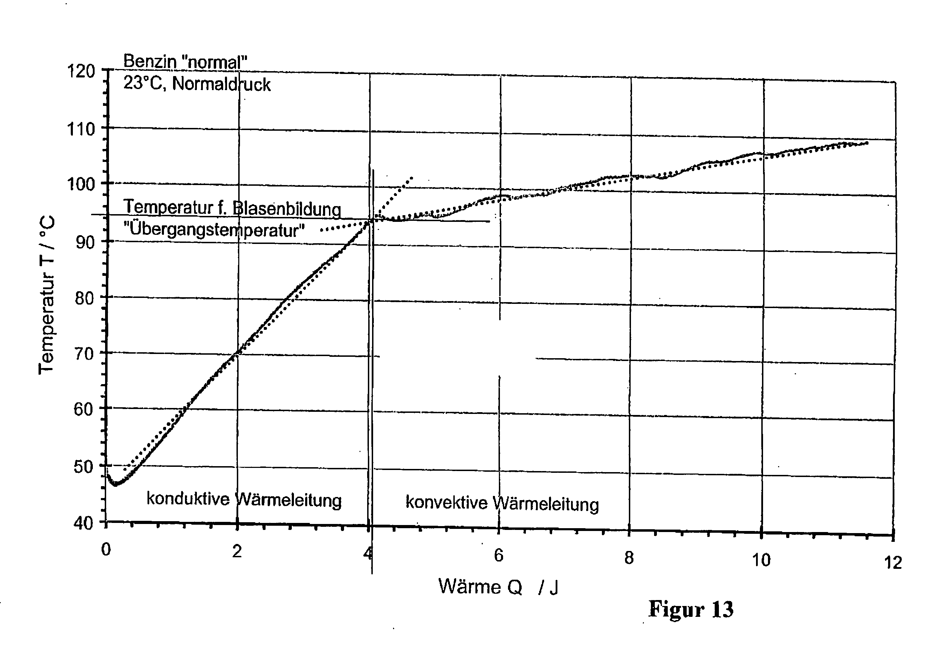

Figur 13 zeigt ebenfalls den Temperaturverlauf an einem in Ottokraftstoff eingetauchten Heizelement, wobei auch hier ein rampenförmiger Heizstromanstieg zugrunde liegt. Allerdings ist hier die Temperatur nicht gegenüber der Zeit aufgetragen, sondern gegenüber der dem Heizelement zugeführten Wärme, welche über eine zeitliche Integration der dem Heizelement zugeführten elektrischen Leistung ermittelt wird. Wie zu erkennen ist, ergeben sich in dieser Darstellung zwei annähernd lineare Bereich mit jedoch sehr unterschiedlicher Steigung. Die Übergangstemperatur wird nun bestimmt, indem für jeden der beiden Bereich eine Ausgleichsgrade bestimmt wird und der Schnittpunkt der beiden Ausgleichsgraden ermittelt wird.

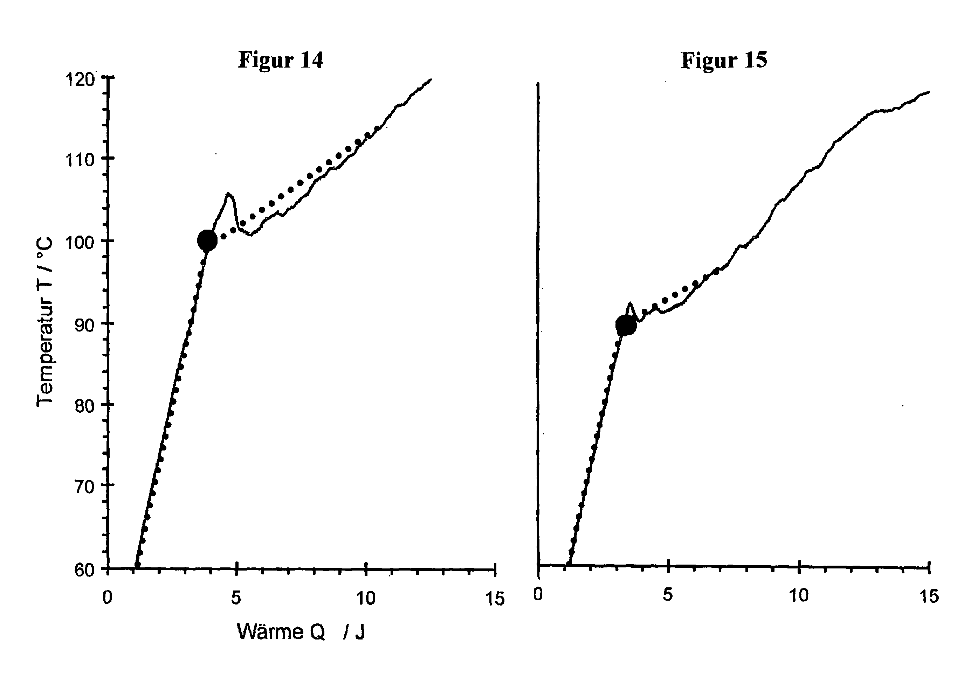

In Figur 14 und 15 sind die entsprechenden Temperaturverläufe für Ottokraftstoff mit Winterqualität (Figur 14) und Sommerqualität (Figur 15) dargestellt. Dabei ergibt sich für Ottokraftstoff mit Winterqualität eine Übergangstemperatur von 100°C und für Ottokraftstoff mit Sommerqualität eine Übergangstemperatur von 90°C.

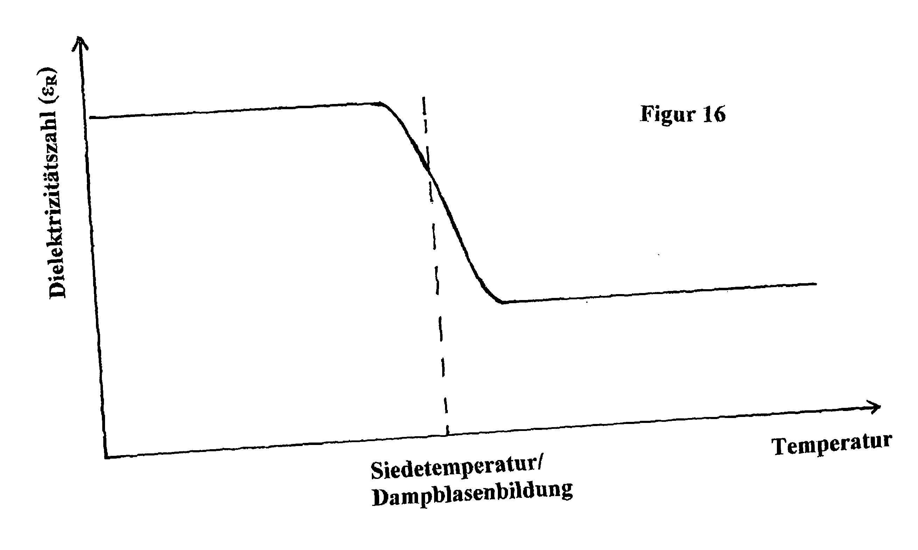

Figur 16 zeigt den Verlauf der Dielektrizitätszahl (ER) in der Nähe des Heizelements in Abhängigkeit von der Temperatur. Wie zu erkennen ist, verringert sich die Dielektrizitätszahl sehr stark mit Beginn der Dampfblasenbildung.

## Patentansprüche

1. Verfahren zur Bestimmung der Verdampfungseigenschaft von Kraftstoff-Flüssigkeiten für Verbrennungsmotoren von Kraftfahrzeugen,
**gekennzeichnet durch** die folgenden Merkmale:
a) die Verwendung eines elektrischen Heizelements (8), das zumindest teilweise in der Flüssigkeit angeordnet ist, um eine Wärmeleitung zwischen dem Heizelement und der Kraftstoff-Flüssigkeit zu bewirken,
b) das Aufheizen des Heizelements (8) zumindest bis zur Bildung von Kraftstoffdampfblasen am Heizelement,
c) die Messung der Temperatur an oder zumindest in unmittelbarer Nähe der Heizelements (8) beim Aufheizen,
d) Detektion der Dampfblasen zur Bestimmung der Siedetemperatur, der Anfangssiedetemperatur der Siedebereichs oder der Siedelinie der Flüssigkeit,
e) wobei zur Detektion der Dampfblasen der Einfluß des dampfblaseninduzierten konvektiven Wärmetransports zwischen Heizelement (8) und Kraftstoff-Flüssigkeit auf den Temperaturverlauf ermittelt wird.

2. Verfahren nach Anspruch 1,
**dadurch gekennzeichnet, dass**
ein elektrisches Heizelement (8) mit einem temperaturabhängigen Widerstand verwendet wird, wobei die Temperaturmessung über eine Bestimmung des temperaturabhängigen Widerstandes erfolgt, so daß das Heizelement selbst als Temperatursensor verwendet wird.

3. Verfahren nach Anspruch 1,
**dadurch gekennzeichnet, dass**
ein vom Heizelement (8) unabhängiger Temperatursensor zur Temperaturmessung verwendet wird.

4. Verfahren nach einem der vorstehenden Ansprüche,
**dadurch gekennzeichnet, dass**
als Heizelement (8) ein Widerstandsheizelement verwendet wird.

5. Verfahren nach Anspruch 4,
**dadurch gekennzeichnet, dass**
mindestens eine der beiden Elektroden des kapazitiven Sensorelements zusätzlich auch als Widerstandsheizelement (8) verwendet wird.

6. Verfahren nach Anspruch 4 oder 5,
**dadurch gekennzeichnet, dass**
das Heizelement (8) zum Aufheizen mit einem zeitlich ansteigenden Strom, insbesondere einem linear ansteigenden Strom, beaufschlagt wird.

7. Verfahren nach Anspruch 4 oder 5,
**dadurch gekennzeichnet, dass**
das Heizelement (8) für eine bestimmte Zeitdauer mit einem konstanten Strom beaufschlagt wird.

8. Verfahren nach Anspruch 7,
**dadurch gekennzeichnet, dass**
das Heizelement (8) im Pulsbetrieb mit Strom beaufschlagt wird.

9. Verfahren nach Anspruch 8,
**dadurch gekennzeichnet, dass**
die Pulsweite der Strompulse moduliert wird.

10. Verfahren nach Anspruch 8 oder 9,
**dadurch gekennzeichnet, dass**
die Stromhöhe der Strompulse variiert wird.

11. Verfahren nach einem der vorstehenden Ansprüche,
**dadurch gekennzeichnet, dass**
die dem Heizelement (8) zugeführte elektrische Wärme über eine zeitliche Integration der dem Heizelement zugeführten elektrischen Leistung ermittelt wird.

12. Verfahren nach Anspruch 1 und 11,
**dadurch gekennzeichnet, dass**
die Ermittlung des Einflusses des dampfblaseninduzierten konvektiven Wärmetransports zwischen Heizelement (8) und Flüssigkeit auf den Temperaturverlauf in Abhängigkeit von der dem Heizelement zugeführten Wärme ermittelt wird.

13. Verfahren nach einem der vorstehenden Ansprüche,
**dadurch gekennzeichnet, dass**
zum Schutz des Heizelement (8) vor Überhitzung die Zufuhr der elektrischen Leistung zum Heizelement bei Erreichen einer vorbestimmten Temperatur verringert oder unterbunden wird.

14. Einrichtung zur Bestimmung der Verdampfungseigenschaft von Kraftstoff-Flüssigkeiten für Verbrennungsmotoren von Kraftfahrzeugen,
**gekennzeichnet durch**
- ein elektrischen Heizelements (8), das zum Aufheizen der Kraftstoff-Flüssigkeit zumindest teilweise in derselben angeordnet ist
- Mittel zur Messung der Temperatur an oder zumindest in unmittelbarer Nähe des Heizelements ,
- einen mit Flüssigkeit gefüllten Meßraum zur Bewirkung einer dampfblaseninduzierten Strömung entlang des Heizelements (8), wobei der Messraum ein Außenrohr (14)und ein darin angeordnetes Innenrohr (16) umfasst, in dem das Heizelement angeordnet ist.

15. Einrichtung nach Anspruch 14,
**dadurch gekennzeichnet, dass**
die Längsachse des Außenrohrs (14) und des Innenrohrs (16) im wesentlichen lotrecht im Kraftfahrzeug angeordnet ist.

16. Einrichtung nach Anspruch 15,
**dadurch gekennzeichnet, dass** die Heizfläche des Heizelements (8) im wesentlichen parallel zum Lot angeordnet ist.

17. Einrichtung nach einem der Ansprüche 14 bis 16,
**dadurch gekennzeichnet, dass**
die freie Querschnittsfläche (18) des Außenrohrs mindestens dreimal so groß ist wie die frei Querschnittsfläche (19) des Innenrohrs.

18. Einrichtung nach einem der Ansprüche 14 bis 17,
**dadurch gekennzeichnet, dass**
die Höhe (Hᵢ) des Innenrohrs mindestens eineinhalbmal so groß ist wie die Erstreckung (L) der Heizfläche des Heizelements (8) in Längsrichtung des Innenrohrs,

19. Einrichtung nach einem der Ansprüche 14 bis 18,
**dadurch gekennzeichnet, dass**
die Höhe (Hₐ) des Außenrohrs mindestens so groß ist wie die Höhe (Hᵢ) des Innenrohrs plus dem freien Durchmesser des Innenrohrs.

20. Einrichtung nach einem der Ansprüche 14 bis 19,
**dadurch gekennzeichnet, dass**
der Messraum in dem Abzweig (7) eines T-förmigen Leitungsabschnitts der Kraftstoffleitung angeordnet ist.

21. Einrichtung nach Anspruch 20,
**dadurch gekennzeichnet, dass**
der Abzweig (7) des T-förmigen Leitungsabschnitts das Außenrohr bildet.

22. Einrichtung nach einem der Ansprüche 14 bis 21,
**dadurch gekennzeichnet, dass**
das Außenrohr (14) und/oder das Innenrohr (16) an zumindest einer Stirnseite eine gelochte Metallplatte oder ein Metalldrahtnetz (26) als Flammschutz aufweist.

23. Einrichtung nach Anspruch 15,
**gekennzeichnet durch**
Mittel (26), die eine schnelles Herausfließen von Kraftstoff aus dem das Heizelement (8) enthaltenen Innenrohr (16) in einer extremen Schräglage des Kraftfahrzeuges oder bei einem Überschlag des Kraftfahrzeuges verhindern.

24. Einrichtung nach Anspruch 23 und 22,
**dadurch gekennzeichnet, dass** das Mittel (26) von der gelochten Metallplatte oder dem Metalldrahtnetz gebildet ist.

25. Einrichtung nach einem der Ansprüche 14 bis 24,
**dadurch gekennzeichnet, dass**
ein elektrisches Heizelement (8) mit einem temperaturabhängigen Widerstand verwendet wird, wobei die Temperaturmessung über eine Bestimmung des temperaturabhängigen Widerstandes erfolgt, so daß das Heizelement selbst als Mittel zur Temperaturmessung verwendet wird.

26. Einrichtung nach einem der Ansprüche 14 bis 25,
**gekennzeichnet durch**
ein kapazitives Sensorelement zur Messung der dielektrischen Eigenschaften des von dem Heizelement (8) aufgeheizten Kraftstoffs.

## Claims

1. Method for determining the evaporation property of fuel liquids for combustion engines in motor vehicles,
**characterized by** the following features:
a) the use of an electrical heating element (8) that is located at least in part in the liquid, in order to effect heat conduction between the heating element and the fuel liquid,
b) he heating of the heating element (8) at least until fuel vapor bubbles are formed on the heating element,
b) the measurement of temperature at or at least directly near the heating element (8) as this is heated up,
c) detection of the vapor bubbles to determine the boiling temperature, the initial bubble-point temperature of the boiling range or the liquid's bubble-point curve
d) hereby the influence of the vapor bubble induced convective heat transport between the heating element (8) and the fuel liquid on the temperature curve is determined for detection of the vapor bubbles.

2. Method according to claim 1,
wherein
an electrical heating element (8) with a temperature-dependent resistor is used, whereby temperature measurement takes place by determining the temperature-dependent resistance, so that the heating element itself is used as a temperature sensor.

3. Method according to claim 1,
wherein
a temperature sensor independent of the heating element (8) is used for temperature measurement.

4. Method according to one of the above claims, wherein
a resistor heating element is used as a heating element (8).

5. Method according to claim 4,
wherein
at least one of the two electrodes of the capacitive sensor element is additionally used as a resistance heating element (8).

6. Method according to claim 4 or 5,
wherein
the heating element (8) is heated by having a current applied over time, particularly a current that increases linearly.

7. Method according to claim 4 or 5,
wherein
the heating element (8) has a constant current applied for a certain time period.

8. Method according to claim 7,
wherein
the heating element (8) has current applied in pulsed mode.

9. Method according to claim 8,
wherein
the pulse width of the current pulses is modulated.

10. Method according to claim 8 or 9, wherein the current
quantity of the current pulses is varied.

11. Method according to one of the above claims,
wherein
the electrical heat supplied to the heating element (8) is determined by timed integration of the electrical power supplied to the heating element.

12. Method according to claim 1 and 11,
wherein
the determination of the influence of the vapor bubble-induced convective heat transport between the heating element (8) and liquid on the temperature curve is established depending on the heat supplied to the heating element.

13. Method according to one of the above claims,
wherein
the supply of electrical power to the heating element is reduced or stopped at a pre-determined
temperature in order to protect the heating element (8) from overheating.

14. Equipment for determining the evaporation property of fuel liquids
for combustion engines in motor vehicles,
**characterized by**
- an electrical heating element (8) that is situated at least partly within the fuel liquid in order to heat it
- means of measuring temperature at or at least in the direct vicinity of the heating element
- a measuring space filled with liquid to effect a vapor bubble-induced flow along the heating element (8), whereby the measuring space comprises an outside pipe (14) and an inside pipe (16) within it, where the heating element is located.

15. Equipment according to claim 14,
wherein
the longitudinal axis of the outside pipe (14) and the inside pipe (16) is essentially arranged perpendicularly in the motor vehicle.

16. Equipment according to claim 15,
wherein
the heating area of the heating element (8) is essentially arranged parallel to the perpendicular.

17. Method according to one of the claims 14 to 16,
wherein
the free cross-section area (18) of the outside pipe is at least three times as large as the free cross-section area (19) of the inside pipe.

18. Method according to one of the claims 14 to 17,
wherein
the height (Hᵢ) of the inside pipe is at least one and a half times as large as the distance (L) between the heating area of the heating element (8) in the longitudinal direction of the inside pipe.

19. Equipment according to one of the claims 14 to 18,
wherein
the height (Hₐ) of the outside pipe is at least as large as the height (Hᵢ) of the inside pipe plus the free diameter of the inside pipe.

20. Equipment according to one of the claims 14 to 19,
wherein
the measuring space is located in the branch (7) of a T-shaped pipe section of the fuel pipe.

21. Equipment according to claim 20,
wherein
the branch (7) of the T-shaped pipe section forms the outside pipe.

22. Equipment according to one of the claims 14 to 21,
wherein
the outside pipe (14) and/or the inside pipe (16) has a perforated metal plate or metal wire meshing (26) on at least one narrow end as a flame retardant.

23. Equipment according to claim 15,
**characterized by**
means (26) which prevent fuel flowing fast out of the inside pipe (16) containing the heating element (8) when the motor vehicle is in an extremely sloped position or when the vehicle rolls over.

24. Equipment according to claim 23 and 22,
wherein
the means (26) is formed by the perforated metal plate or the metal wire meshing.

25. Equipment according to one of the claims 14 to 24,
wherein
an electrical heating element (8) with a temperature-dependent resistor is used, whereby temperature measurement takes place by determining the temperature-dependent resistance, so that the heating element itself is used as a means of temperature measurement.

26. Equipment according to one of the claims 14 to 25,
**characterized by**
a capacitive sensor element for the measurement of dielectric features of the fuel heated by the heating element (8).

## Revendications

1. Procédé de détermination de la propriété de vaporisation de carburants liquides pour les moteurs à combustion de véhicules
**caractérisé par** les caractéristiques suivantes :
a) l'utilisation d'un élément chauffant électrique (8), qui est disposé au moins en partie dans le liquide, afin de produire une conduction thermique entre l'élément chauffant et le carburant,
b) le chauffage de l'élément chauffant (8) au moins jusqu'à la formation de bulles de vapeur de carburant sur l'élément chauffant,
c) la mesure de la température sur ou au moins à proximité immédiate de l'élément chauffant (8) lors du chauffage,
d) la détection des bulles de vapeur pour déterminer la température d'ébullition, la température d'ébullition initiale de la plage d'ébullition ou la ligne d'ébullition du liquide,
e) l'influence du transfert de chaleur convectif induit par les bulles de vapeur entre l'élément chauffant (8) et le carburant liquide sur l'évolution de la température étant déterminée pour détecter les bulles de vapeur.

2. Procédé selon la revendication 1, **caractérisé en ce qu'**un élément chauffant électrique (8) avec une résistance à dépendance thermique est utilisé, la mesure de température s'effectuant par l'intermédiaire de la résistance à dépendance thermique, si bien que l'élément chauffant même est utilisé comme capteur de température.

3. Procédé selon la revendication 1, **caractérisé en ce qu'**un capteur de température indépendant de l'élément chauffant (8) est utilisé pour la mesure de température.

4. Procédé selon l'une quelconque des revendications précédentes, **caractérisé en ce qu'**un élément chauffant à résistance est utilisé comme élément chauffant (8).

5. Procédé selon la revendication 4, **caractérisé en ce qu'**au moins une des deux électrodes de l'élément sensible capacitif est également utilisé en supplément comme élément chauffant à résistance (8).

6. Procédé selon la revendication 4 ou 5, **caractérisé en ce que** l'élément chauffant (8) est alimenté par un courant croissant dans le temps, en particulier un courant augmentant de façon linéaire, pour produire le chauffage.

7. Procédé selon la revendication 4 ou 5, **caractérisé en ce que** l'élément chauffant (8) est alimenté par un courant constant pendant une durée déterminée.

8. Procédé selon la revendication 7, **caractérisé en ce que** l'élément chauffant (8) est alimenté en courant en mode pulsé.

9. Procédé selon la revendication 8, **caractérisé en ce que** la largeur des impulsions de courant est modulée.

10. Procédé selon la revendication 8 ou 9, **caractérisé en ce que** l'intensité des impulsions de courant varie.

11. Procédé selon l'une quelconque des revendications précédentes, **caractérisé en ce que** la chaleur électrique transmise à l'élément chauffant (8) est déterminée en réalisant une intégration temporelle de la puissance électrique transmise à l'élément chauffant.

12. Procédé selon les revendications 1 et 11, **caractérisé en ce que** l'influence du transfert de chaleur convectif induit par les bulles de vapeur entre l'élément chauffant (8) et le liquide sur l'évolution de la température est déterminée en fonction de la chaleur transmise à l'élément chauffant.

13. Procédé selon l'une quelconque des revendications précédentes, **caractérisé en ce que** pour protéger l'élément chauffant (8) contre toute surchauffe, l'arrivée de la puissance électrique à l'élément chauffant est réduite ou interrompue lorsqu'une température prédéfinie est atteinte.

14. Dispositif de détermination de la propriété de vaporisation de carburants liquides pour les moteurs à combustion de véhicules
**caractérisé par**
- un élément chauffant électrique (8), qui est disposé au moins en partie dans le carburant liquide pour chauffer celui-ci,
- un moyen de mesure de la température sur ou au moins à proximité immédiate de l'élément chauffant,
- un espace de mesure rempli de liquide, permettant de produire un écoulement induit par les bulles de vapeur le long de l'élément chauffant (8), l'espace de mesure comprenant un tube extérieur (14) et un tube intérieur (16) disposé dedans, dans lequel tube intérieur est placé l'élément chauffant.

15. Dispositif selon la revendication 14, **caractérisé en ce que** l'axe longitudinal du tube extérieur (14) et du tube intérieur (16) est disposé dans le véhicule quasiment à la verticale.

16. Dispositif selon la revendication 15, **caractérisé en ce que** la surface chauffante de l'élément chauffant (8) est disposée quasiment parallèlement à la ligne verticale.

17. Dispositif selon l'une quelconque des revendications 14 à 16, **caractérisé en ce que** la section libre (18) du tube extérieur est au moins trois plus grande que la section libre (19) du tube intérieur.

18. Dispositif selon l'une quelconque des revendications 14 à 17, **caractérisé en ce que** la hauteur (Hᵢ) du tube intérieur est au moins une fois et demie plus grande que l'étendue (L) de la surface chauffante de l'élément chauffant (8) dans le sens longitudinal du tube intérieur.

19. Dispositif selon l'une quelconque des revendications 14 à 18, **caractérisé en ce que** la hauteur (Hₐ) du tube extérieur est au moins aussi grande que la hauteur (Hᵢ) du tube intérieur plus le diamètre libre du tube intérieur.

20. Dispositif selon l'une quelconque des revendications 14 à 19, **caractérisé en ce que** l'espace de mesure est disposé dans la dérivation (7) d'une section en forme de T de la conduite de carburant.

21. Dispositif selon la revendication 20, **caractérisé en ce que** la dérivation (7) de la section de conduite en forme de T constitue le tube extérieur.

22. Dispositif selon l'une quelconque des revendications 14 à 21, **caractérisé en ce que** le tube extérieur (14) et/ou le tube intérieur (16) présente sur au moins une face frontale une plaque métallique perforée ou un treillis en fil métallique (26) servant de pare-flammes.

23. Dispositif selon la revendication 15, **caractérisé par** des moyens (26) qui empêchent un écoulement rapide de carburant depuis le tube intérieur (16) contenant l'élément chauffant (8) dans une position inclinée extrême du véhicule ou dans le cas d'un retournement du véhicule.

24. Dispositif selon les revendications 22 et 23, **caractérisé en ce que** le moyen (26) est formé par la plaque métallique perforée ou le treillis en fil métallique.

25. Dispositif selon l'une quelconque des revendications 14 à 24, **caractérisé en ce qu'**un élément chauffant électrique (8) avec une résistance à dépendance thermique est utilisé, la mesure de température s'effectuant par l'intermédiaire de la résistance à dépendance thermique, si bien que l'élément chauffant même est utilisé comme moyen de mesure de température

26. Dispositif selon l'une quelconque des revendications 14 à 25, **caractérisé par** un élément sensible capacitif permettant de mesurer les propriétés diélectriques du carburant chauffé par l'élément chauffant (8).
